# NEUE EUROPÄISCHE PATENTSCHRIFT

(11) **EP 0 854 709 B2**
(45) Veröffentlichungstag und Bekanntmachung des Hinweises auf die Entscheidung über den Einspruch: **23.02.2005**
(45) Hinweis auf die Patenterteilung: 11.04.2001
(21) Anmeldenummer: 96929309.1
(22) Anmeldetag: 22.08.1996
(51) Int. Cl.: A61K 31/19, A61K 35/78

(54) **VERWENDUNG VON BOSWELLIASÄURE UND IHREN DERIVATEN ZUR HEMMUNG DER NORMALEN UND GESTEIGERTEN LEUKOZYTENELASTASE- ODER PLASMINAKTIVITÄT**
USE OF BOSWELLIC ACID AND ITS DERIVATIVES FOR INHIBITING NORMAL AND INCREASED LEUCOCYTIC ELASTASE OR PLASMIN ACTIVITY
UTILISATION D'ACIDE DE BOSWELL ET DE SES DERIVES POUR INHIBER L'ACTIVITE NORMALE OU ACCRUE DE L'ELASTASE LEUCOCYTAIRE OU DE LA PLASMINE

(30) Priorität: 23.08.1995 DE 19531067
(43) Veröffentlichungstag der Anmeldung: 29.07.1998
(73) Patentinhaber: AMMON, Hermann P.T., 72072 Tübingen (DE)
(72) Erfinder: AMMON, Hermann, P., T., D-72072 Tübingen (DE); SAFAYHI, Hasan, D-72076 Tübingen (DE)
(74) Vertreter: Hiebl, Inge, Dr.
(86) Internationale Anmeldenummer: PCT/EP1996/003705
(87) Internationale Veröffentlichungsnummer: WO 1997/007796

(56) Entgegenhaltungen:
- EP-A- 0 431 076
- EP-A- 0 552 657
- WO-A-96/19212
- INT. J. IMMUNOPHARMACOL., Bd. 14, Nr. 7, 1992, Seiten 1139-1143, XP000570505 A. KAPIL ET AL.: "Anticomplementary activity of boswellic acid: an inhibitor of C3-convertase of the classical complement pathway."
- INT. J. IMMUNOPHARMACOL., Bd. 11, Nr. 6, 1989, Seiten 647-652, XP000572594 M.L. SHARMA ET AL.: "Anti-arthritic activity of boswellic acids in bovine serum albumin(BSA)-induced arthritis."
- ANN. REV. MED., Bd. 36, 1985, Seiten 207-216, XP000570622 A. JANOFF: "Elastase in tissue injury." in der Anmeldung erwähnt
- Bilateral Seminars of the International Bureau, Vol. 23: "Indo-German Workshop on Antiinflammatory Drugs from Natural Scources", herausgegeben vom Forschungszentrum Jülich
- Pschyrembel, 258, Auflage, S. 107/108, 136, 233, 945/946
- Ying et al., "Inhibition of Human Leukocyte Elastase by Ursolic acid: Evidence for a binding site for pentacyclic triterpenes", 1991, Biochem. J., 277:521-526
- Reddy et al., "Studies on the Metabolism of Glycosaminoglycans under the Influence of new herbal anti-inflammatory Agents", 1989, Biochem Pharm., 38(20):3527-3534
- Sharma et al., "Effect of a Salai guggal ex Boswellia serrata on cellular and humoral immune responses and leukocyte migration", 1988, Agents and Actions, 24(1/2):161-164
- Planta Med., 59, 1993, S. 12-16; M. Duviejua at al.: "Anti-inflammatory Activity of Resins from some Species of the Plant Familiy Burseraceae"
- Chinese Medical Science Journal, 7(1), 1992, S. 12-15; J. Yongkui et al.: "Growth inhibition and differentiation of promyelotic cells (HL-60) induced by BC-4, an active principal from Boswellia Carterii Birdw"
- Carbohydrate Res., 223, 1992, S. 321-327; A.K. Sen et al.: "Isolation and structure of a 4-O-methyl-glucuronoarabinogalactan from Boswellia serrata
- Jour. of Ethnopharmacology, 33, 1991, S. 91-95; R.R. Kulkarni et al.: "Treatment of osteoarthritis with a herbomineral formulation: a double-blind, placebo-controlle, cross-over study"
- Planta Medica, Bd. 19, 1970-71, S. 333-341; M.K. Menon et al.: "Analgesic and psychopharmacological effects of the gum resin of Boswellia serrata"
- Life Science, Vol. 8, Part I, 1969, S. 1023-1028; A. Kar et al.: "Analgesic effects of the gum resin of Boswellia serrata ROXB"

## Beschreibung

Die Erfindung betrifft ebenfalls die Verwendung reiner Boswelliasäure oder eines physiologisch annehmbaren Salzes, eines Derivats, eines Salzes des Derivats oder einer Boswelliasäure enthaltenden pflanzlichen Zubereitung zur Herstellung eines Arzneimittels für die Behandlung der Zerstörung von Funktionsgewebe bei Krankheiten, die durch gesteigerte Leukozytenelastaseoder Plasminaktivität hervorgerufen werden bzw. die durch Hemmung von normaler Leukozytenelastase- oder Plasminaktivität behandelbar sind, in der Human- oder Veterinärmedizin.

Erfindungsgemäß erfolgt die Verwendung insbesondere bei Lungenemphysem, akutem Atemnotsyndrom, Schocklunge, zystischer Fibrose (Mucoviscidose), chronischer Bronchitis, Glomerulonephritis und rheumatischer Arthritis, die durch gesteigerte Leukozytenelastaseaktivität hervorgerufen werden, Tumoren und Tumorenmetastasen, die durch gesteigerte Plasminaktivität hervorgerufen werden bzw. die durch Hemmung von normaler Leukozytenelastase- oder Plasminaktivität behandelbar sind.

Die EP-A-552 657 offenbart die Verwendung von Boswelliasäuren zur Prophylaxe oder Behandlung von Entzündungserkrankungen, die mit einer gesteigerten Leukotrienbildung einhergehen. Diese Entgegenhaltung erwähnt jedoch nicht den Zusammenhang einer Leukozytenelastase- oder Plasminaktivität im Zusammenhang mit Krankheiten wie etwa der zystischen Fibrose oder der chronischen Bronchitis.

Int. J. Immunopharmacol., 14(7), 1992, S. 1139-1143, Kapil, A. et al., beschreibt die inhibierende Wirkung der Boswelliasäure auf das Komplementsystem, was sich letztlich als generell entzündungshemmend auswirkt. Der Zusammenhang von Leukozytenelastase- und Plasminaktivität mit konkreten Krankheitserscheinungen und deren erfolgreiche Behandlung mit Boswelliasäure wird hier jedoch nicht angesprochen.

Int. J. Immunopharmacol. 11(6), 1989, S. 647-652, Sharma, M.L., et al., weist auf Boswelliasäure als vielversprechendes Mittel gegen Arthritis hin. Die orale Verabreichung von Boswelliasäuren soll dabei eine Verringerung der Leukozytenmenge bedingen. Es wird jedoch keinerlei Zusammenhang zwischen Leukozytenelastase/Plasmin und Boswelliasäure hergestellt.

Ann. Rev. Med. 36, 1985, S. 207-216, Janoff, A., charakterisiert die Rolle der Elastase aus neutrophilen Granulozyten beim Abbau von Gewebe und hebt die Problematik bei einer unkontrollierten Freisetzung dieses Enzyms mit der damit verbundenen Notwendigkeit von spezifischen Elastaseinhibitoren hervor. Die Eignung der Boswelliasäure zur Lösung dieser Problematik wird jedoch nicht angesprochen.

### LEUKOZYTENELASTASE

### Vorkommen und biologische Aktivität der humanen Leukozytenelastase

Die humane Leukozytenelastase (HLE; EC 3.4.21.37) ist ein Glykoprotein mit Protease-Aktivität. Sie wird in neutrophilen Granulozyten (PMNL) des Menschen in inaktiver Form gespeichert, bei Aktivierung der neutrophilen Granulozyten aus Granula dieser Zellen freigesetzt und katalysiert dann als Enzym (Serinprotease) den proteolytischen Abbau von Elastin, Kollagen, Fibronectin und weiteren Proteinen.

### Pathophysiologische Bedeutung

Die Serinprotease-Aktivität der humanen Leukozytenelastase ist - im Verein mit anderen Entzündungsmediatoren - beim Entstehen und bei der Aufrechterhaltung krankhafter Veränderungen und der Verschlimmerung solcher Vorgänge - insbesondere durch den Abbau von Bestandteilen des Stützgerüstes vieler Organe und Gewebe - mitbeteiligt. Insofern wird der humanen Leukozytenelastase bei folgenden Erkrankungen des Menschen eine Rolle zugeschrieben (für eine Übersicht: vgl. Janoff, Annu. Rev. Med. 36: 207-216, 1985):
- Lungenemphysem, akutes Atemnotsyndrom, Schocklunge,
- zystische Fibrose (Mucoviscidose),
- chronische Bronchitis,
- Glomerulonephritis,
- rheumatische Arthritis.

Allgemein wird eine Beteiligung von humaner Leukozytenelastase bei katabolen Prozessen von Entzündungen verschiedener Genese, die mit einer neutrophilen Granulozyten-Infiltration einhergehen, postuliert. Aus Grundlagenbefunden mit isolierten Zellen kann ferner eine Rolle bei der Endotoxin-ausgelösten, durch neutrophile Granulozyten vermittelten Leberschädigung gefolgert werden (Sauer et al., Naunyn-S. Arch. Pharmacol. 351 S: Abstr. 495, 1995).

### Inhibitoren der humanen Leukozytenelastase

Es sind eine Vielzahl von natürlich vorkommenden und synthetischen Inhibitoren für die humane Leukozytenelastase bekannt (Groutas 1987, Med. Res. Rev. 7: 227-241; Bode et al. 1989, Biochemistry 28: 1951-1963). Die Wirksamkeit einiger dieser Verbindungen in experimentellen Tiermodellen ist gezeigt (Powers 1983, Am. Rev. Respir. Dis. 127: S. 54-S. 58; Schnebli 1985, in Handbook of Inflammation, Eds: Bonta, Bray & Parnham, Vol. 5: 321-333, Elsevier Sci. Publ., Amsterdam; Soskel et al. 1986, Am. Rev. Respir. Dis. 133: 635638). Die Hemmung der humanen Leukozytenelastase durch einige pentacyclische Triterpenderivate wurde gezeigt. Die Wirksamkeit der einzelnen Derivate war dabei unterschiedlich (Ki-Werte 4 bis 185 µM). Am wirksamsten war Ursolsäure (Ki = 4 bis 6 *µ*M). Die Boswelliasäure-Reihe wurde in dieser Studie nicht getestet (Ying et al. 1991, Biochem. J. 277: 521-526).

### Methode zur Messung der humanen Leukozytenelastase-Aktivität in vitro

Die Aktivität wurde mit reiner humaner Leukozytenelastase (Calbiochem) und dem Substrat MeO-Suc-Ala-Ala-Pro-Val-p-Nitroanilid in Dulbecco's phosphatgepufferter Salzlösung (DPBS) in Gegenwart von 10%igem Dimethylsulfoxid (DMSO) photometrisch bei Raumtemperatur und 405 nm bestimmt (Bieth et al. 1974, Biochem. Meth. 11: 350-357).

### Methode zur Messung der Chymotrypsin (CT) Aktivität in vitro

Die Wirkung von Acetyl-11-keto-β-Boswelliasäure (AKBA) auf die reine Chymotrypsin-Aktivität (Sigma) wurde ebenfalls bestimmt, um eine eventuelle unselektive Hemmung verschiedener Serinproteasen zu untersuchen. Der Test wurde mit MeO-Suc-Ala-Ala-Pro-Phe-p-Nitroanilid als Substrat in Dulbecco's phosphatgepufferter Salzlösung (DPBS) in Gegenwart von 10%igem Dimethylsulfoxid photometrisch bei Raumtemperatur und 410 nm durchgeführt.

### Ergebnisse der Hemmung der Leukozytenelastaseaktivität durch Boswelliasäuren

In einem etablierten in vitro Testsystem wurden die Aktivitäten zweier Serinprotease-Enzyme (humane Leukozytenelastase und Chymotrypsin) gemessen. Es wurden reine Enzyme von Calbiochem bzw. SIGMA eingesetzt und die Enzymaktivität als die Geschwindigkeit bestimmt, mit der die Enzyme aus ihren Substraten pro Zeiteinheit durch Hydrolyse p-Nitroanilin freisetzten. Es wurden für die HLE-Aktivitätsbestimmungen drei Meßreihen bei drei verschiedenen Substratkonzentrationen durchgeführt. Die 100 %-Kontrollen enthielten keine Testsubstanzen, sondern nur die entsprechende Lösungsmittelmenge. Die Effekte von verschiedenen Konzentrationen an Acetyl-11-keto-β-Boswelliasäure (AKBA) auf die humane Leukozytenelastase in diesen drei Meßreihen werden als Prozent der 100 %-Kontrollen als Mittelwerte + S.D. von jeweils drei unabhängigen Messungen gezeigt.

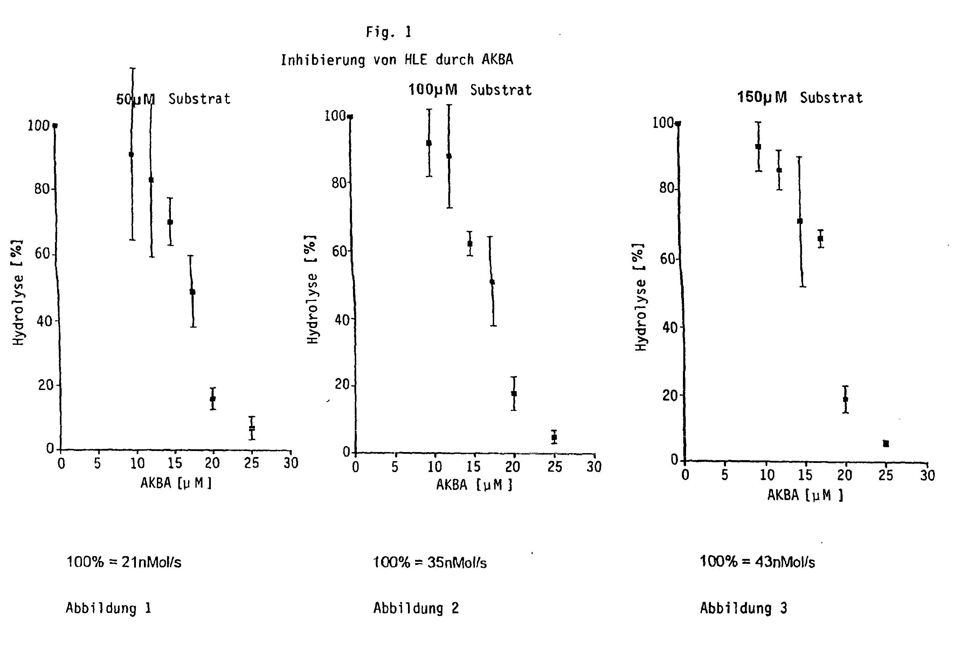

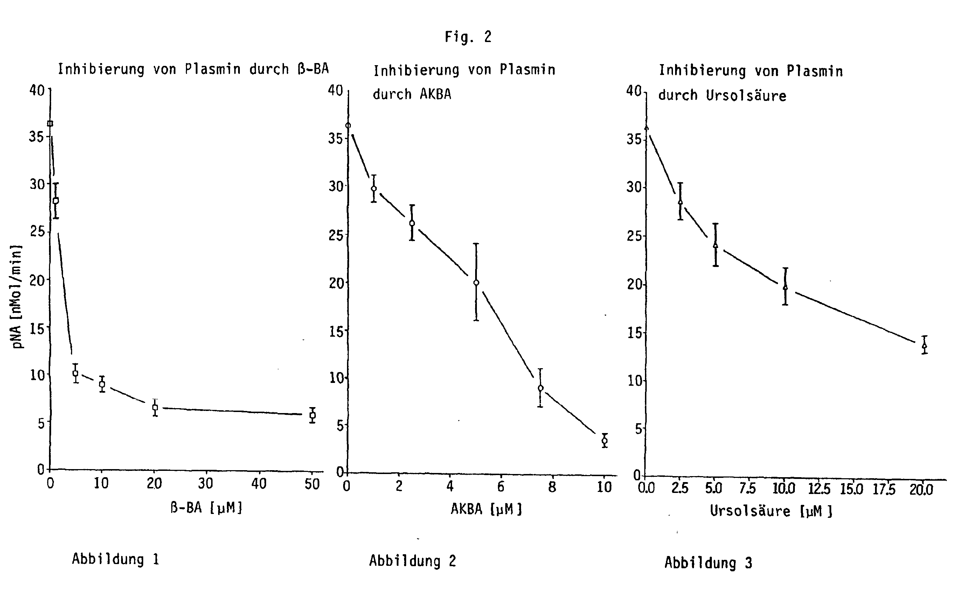

1. Acetyl-11-keto-β-Boswelliasäure hemmte die humane Leukozytenelastase konzentrationsabhängig mit IC50 Werten von ca. 17 µM (bei Substratkonzentrationen von 50, 100 und 150 µM). Bei 20 µM Acetyl-11-keto-β-Boswelliasäure verblieb eine Restaktivität von 18 %; bei 20 µM Ursolsäure betrug die Restaktivität 11 % (vgl. Fig. 1).

2. Keinen Hemmeffekt bei einer Konzentration von jeweils 20 *µ*M im Test besaßen in diesem System folgende als Referenzsubstanzen eingesetzten Verbindungen: Als pentacyclisches Triterpenderivat die Glycerrhitinsäure, als reduzierende bzw. kompetitive 5-Lipoxygenase-Inhibitoren NDGA, MK886 und ICI230,487, als Steroide Cortisol und Testosteron, und als mehrfach ungesättigte langkettige Fettsäure die Arachidonsäure.

3. Keinen signifikanten Hemmeffekt auf die Chymotrypsin-Aktivität besaß Acetyl-11-keto-β-Boswelliasäure (AKBA) in Konzentrationen von 50 und 100 µM (89 und 85 der Kontrollaktivität), während die Ursolsäure die Aktivität von Chymotrypsin bei 50 *µ*M auf 56 % und bei 100 µM auf 30 % der Kontrollaktivität von Chymotrypsin hemmte.

### Zusammenfassung der Ergebnisse für die Hemmung der humanen Leukozytenelastase

Aus diesen Ergebnissen folgt, daß Acetyl-11-keto-β-Boswelliasäure neben ihrer 5-Lipoxygenase-hemmenden Eigenschaft und der damit zusammenhängenden Reduktion der Leukotrien-Biosynthese auch eine Hemmwirkung auf die humane Leukozytenelastase besitzt.

Die Hemmung dieses Enzyms ist deshalb von Bedeutung, weil während der pathophysiologischen Prozesse der zuvor genannten Erkrankungen (Lungenenphysem, akutes Atemnotsyndrom, Schocklunge, chronische Bronchitis, zystische Fibrose, Glomerulonephritis und rheumatische Arthritis) die aus den aktivierten neutrophilen Granulozyten freigesetzte humane Leukozytenelastase eine wichtige Rolle bei der Zerstörung von Funktionsgewebe spielt und somit verantwortlich ist für die durch diese Erkrankungen hervorgerufenen Schäden in der Lunge, in den Nieren und in den Gelenken. Deshalb sollte es möglich sein, durch eine Hemmung der humanen Leukozytenelastase mit Boswelliasäuren oder ihren Derivaten die Schädigung der Organe infolge dieser Erkrankungen zu verhindern. Selektive Hemmstoffe der humanen Leukozytenelastase sind bis heute nicht verfügbar. Nicht-selektive Hemmstoffe eignen sich für die Pharmakotherapie jedoch nicht, da sie durch die Hemmung auch anderer Proteasen gravierende unerwünschte Wirkungen hervorrufen können. Boswelliasäuren und Derivate werden darüber hinaus gut resorbiert und erwiesenermaßen nicht toxisch. Ein weiterer Vorteil beim Einsatz von Boswelliasäuren besteht ferner darin, daß die synchrone Hemmung zweier entzündungsfördernder Mediatorsysteme der Leukozyten durch diese Monosubstanz synergistisch für die Pharmakotherapie einer Reihe von heute ungenügend beherrschbarer Erkrankungen ausgenützt werden könnte. Andere pentacyclische Triterpene sind zwar ebenfalls in der Lage, die HLE zu hemmen (z.B. Ursolsäure), bis auf die Boswelliasäuren besitzen andere pentacyclische Triterpene jedoch keinen Effekt auf die Leukotrien-Biosynthese (Safayhi et al. 1995, Mol. Pharmacol. in press). Wie in der Klasse der Dioxygenasen für die 5-Lipoxygenase (Safayhi et al. JPET 1992) besitzt AKBA auch unter den Serinproteasen eine gewisse selektive Hemmwirkung für die HLE, wie es die fehlende Wirkung auf Chymotrypsin, ein Verdauungsenzym aus der Klasse der Serinproteasen, zeigt.

### PLASMIN

### Vorkommen und biologische Aktivität

Plasmin (EC 3.4.21.7.) ist wie die humane Leukozytenelastase eine Serinprotease. Plasmin katalysiert als Enzym die Hydrolyse von Peptidbindungen, an denen Arginin und Lysin beteiligt sind, und damit den Abbau einer Reihe von Proteinen und Peptiden. Plasmin kommt im Blut als inaktive Vorstufe Plasminogen vor und wird durch proteolytische Aktivierung aus der Vorstufe (Plasminogen) gebildet. Die erhöhte Aktivität von Plasmin wird für die Zerstörung von Zellgerüstproteinen im Verlaufe vieler Erkrankungen, aber auch für das invasive Wachstum und Metastasenbildung von bösartigen Tumoren unter Zerstörung von körpereigenem Funktionsgewebe verantwortlich gemacht (Wangh et al., Int. J. Cancer. 1994, 58: 650-657). Darüber hinaus aktiviert Plasmin auch sogenannte Wachstumsfaktoren, die ebenfalls die Vermehrung von Tumoren stimulieren können (Campbell et al., J. Cell. Physiol. 1994, 159: 1-10). Deshalb erscheint es möglich, durch Hemmung der Plasmin-Aktivität das Wachstum und die Metastasenbildung vieler Krebsarten zu hemmen.

### Hemmung der Plasminaktivität durch Boswelliasäuren

### Methode: Messung der Plasmin-Aktivität

Die Aktivität von humanem Plasmin (SIGMA) wurde in vitro mit dem Substrat D-Ile-Phe-Lys-pNA photometrisch bestimmt (Cs-Szabo et al., Thrombosis Res. 1980, 20: 199-206). Die Plasminaktivität wird als die Freisetzung von p-Nitroanilin pro Minute (nMol/min) aus dem Substrat als Mittelwerte ± S.D. von jeweils n = 3 Messungen angegeben.

### Ergebnisse

Die pentacyclischen Triterpensäuren aus der Boswelliasäure-Reihe, β-Boswelliasäure (β-BA) und Acetyl-11-keto-β-Boswelliasäure (AKBA), hemmen die Plasminaktivität mit vergleichbarer Wirksamkeit mit halbmaximalen Hemmkonstanten von ca. 4 bis 6 µM (Fig. 2, Abb. 1 und 2). Im Gegensatz zur Hemmung der HLE (humane Leukozytenelastase) ist die Ursolsäure hinsichtlich des Effektes auf die Plasmin-Aktivität deutlich weniger wirksam (IC50 von ca. 15 µM; Fig. 2, Abb. 3), während Amyrin die Plasmin-Aktivität in Konzentrationen bis 50µM nicht signifikant beeinflußt (nicht gezeigt).

### Zusammenfassung

In einem in vitro-Testsystem hemmten β-BA und AKBA die Plasmin-Aktivität in Konzentrationen, die nach oraler Einnahme von Weihrauchextrakten im Blut von Menschen erreicht werden können, nahezu vollständig. Andere pentacyclische Triterpensäuren sind in diesem System entweder wesentlich schwächer (Ursolsäure) oder gar nicht (Amyrin) wirksam.

Da die Pläsmin-Aktivität einen der wesentlichen Mechanismen des bösartigen Tumorwachstums unter Zerstörung von Funktionsgewebe im Wirtsorganismus darstellt, erscheint es wahrscheinlich, daß durch den Einsatz von Boswelliasäuren die Entstehung von Karzinomen und Sarkomen verhindert werden könnte.

Bisher gibt es in der Therapie keine zufriedenstellenden Behandlungsmethoden für Krankheiten wie Lungenemphysem, akutes Atemnotsyndrom, Schocklunge, zystische Fibrose (Mucoviscidose), chronische Bronchitis, Glomerulonephritis und rheumatische Arthritis, Krankheiten, die durch gesteigerte Leukozytenelastaseaktivität hervorgerufen werden, und Tumoren und Tumorenmetastasen, die durch gesteigerte Plasminaktivität hervorgerufen werden.

Chronische Bronchitis, Lungenemphysem, akutes Atemnotsyndrom und die Schocklunge (ARDS) gehören zu den Erkrankungen der Atemwege, die auf verschiedene Ursachen zurückgehen können. Obwohl es sich um verschieden definierte Krankheitsentitäten handelt, weisen sie erhebliche Überschneidungen hinsichtlich pathophysiologischer Vorgänge, diagnostischer Maßnahmen und therapeutischer Ansätze auf. Während im Frühstadium durch Ausschalten der Noxe (z.B. Rauchverbot, Arbeitsplatzwechsel, Antibiotika ...) eine grundlegende Besserung zeitigen können, ist beim Forschreiten der Erkrankung, die mit Zerstörung des Funktionsgewebes einhergeht, in der Regel nur noch die Besserung der Symptome möglich (Verbesserung des Sekretabflusses, Sauerstoff-Beatmung).

Beim Emphysem kann rekombinantes α1-Antitrypsin substituiert werden, wenn ein α1-Antitrypsinmangel (Mangel an körpereigenem Proteaseinhibitor) vorliegt. Bei ARDS: Acute/Adult Respiratory Distress Syndrome (Schocklunge), einer schockartig ablaufenden, akut lebensbedrohlichen Lungenfunktionsstörung, die die häufigste Todesursache bei Patienten, die die Frühphase eines Schocks aus primär nicht-pulmonaler Ursache überlebt haben, darstellt sowie eine gefährliche Komplikation bei traumatisierten und operierten Patienten ist, werden medikamentös Aprotinin, Kortikoiden, Heparin und niedermolekulare Dextrane eingesetzt. Je nach Dauer der Ateminsuffizienz beträgt die Letalität heute trotzdem 30 bis 90 %.

Bei der Zystischen Fibrose (Mucoviscidose) handelt es sich um eine erbliche Stoffwechselanomalie, die durch eine generalisierte Dysfunktion exokriner Drüsen (gesteigerte Produktion und hohe Viskosität des Sekrets der mukösen Drüsen der Bronchien und des Verdauungstraktes) zu schweren Komplikationen im Bereich der Atemwege und im Pankreas führen, die fortschreitend den Abbau von Funktionsgewebe in den betroffenen Organen zur Folge haben. Die Therapie konzentriert sich vornehmlich auf die Verhinderung von Komplikationen durch kontinuierliche Betreuung der Atemwege (Physiotherapie, Inhalationen, frühzeitige und gezielte Antibiose).

Bei Arthritis (Gelenkentzündungen) führen unter Beteiligung hydrolysierender Enzyme (z.B. Leukozytenelastase) ablaufende Prozesse zu degenerativen Kapselveränderungen. Medikamentös wird mit nichtsteroidalen Antirheumatika symptomatisch der Folgeschmerz verhindert. Die Behandlung der ursächlich verantwortlichen Folgeschäden bei primär-entzündlichen Arthriden ist mit Steroiden (kortikoiden) möglich, verbietet sich jedoch aufgrund der gravierenden unerwünschten Nebenwirkungen. Eine direkte Hemmung unter Beteiligung der Leukozytenelastase verlaufender kataboler Prozesse ist heute nicht möglich. Glomerulonephritis ist ein Sammelbegriff sehr verschiedenartiger Nierenerkrankungen mit Entzündungsvorgängen, die zu degenerativen Prozessen mit terminaler Niereninsuffizienz führen können. Eine Kausaltherapie fehlt. Bei manchen Formen können Kortikoide helfen, wobei gravierende unerwünschte Wirkungen in Kauf genommen werden müssen.

Zur Behandlung der oben genannten Krankheiten wird von der pharmazeutischen Industrie fieberhaft nach Leukozytenelastase- und -plasmin-Hemmern gesucht, die untoxisch sind.

Der vorliegenden Erfindung liegt die Aufgabe zugrunde, die Verwendung von Präparaten zur Verfügung zu stellen, die zur Prophylaxe und/oder Bekämpfung von Krankheiten, die mit einer übermäßigen Leukozytenelastaseoder Plasminaktivität einhergehen bzw. die durch Hemmung von normaler Leukozytenelastase- oder Plasminaktivität behandelbar sind, dienen. Die erfindungsgemäß zur Verfügung gestellten Präparate sollen insbesondere in der Lage sein, die oben genannten Krankheiten zu heilen. Die erfindungsgemäß verwendeten Arzneimittel sollen während langer Zeit verabreicht werden können, ohne daß irgendwelche Nebenwirkungen auftreten. Das erfindungsgemäß verwendete Arzneimittel soll untoxisch sein und von den Patienten gut toleriert werden.

Überraschenderweise wurde jetzt gefunden, daß Boswelliasäure, ein physiologisch annehmbares Salz, ein Derivat, ein Salz des Derivats oder eine Boswelliasäure enthaltende pflanzliche Zubereitung für die Prophylaxe und/oder die Bekämpfung von Krankheiten, die durch gesteigerte Leukozytenelastase- oder Plasminaktivität hervorgerufen werden, in der Human- oder Veterinärmedizin verwendet werden kann.

Die Pflanze Boswellia serrata und andere Boswellia-Arten verfügen über Inhaltsstoffe, die in der Lage sind, die Leukozytenelastasebildung und die Leukozytenplasminaktivität selektiv zu inhibieren. Boswellia serrata enthält nach Angaben von Pardhy & Bhattacharyya (Ind. J. Chem., 16B:176-178, 1978) im wesentlichen folgende Inhaltsstoffe: β-Boswelliasäure, Acetyl-β-boswelliasäure, Acetyl-11-keto-β-boswelliasäure, 11-Keto-β-boswelliasäure, über die bisher jedoch keine pharmakologischen Untersuchungen im Bereich einer humanen Leukozytenelastase-Hemmung oder Leukozytenplasmin-Hemmung durchgeführt wurden.

Die Strukturformeln von Boswelliasäure und einigen ihrer Derivate werden im folgenden aufgeführt:

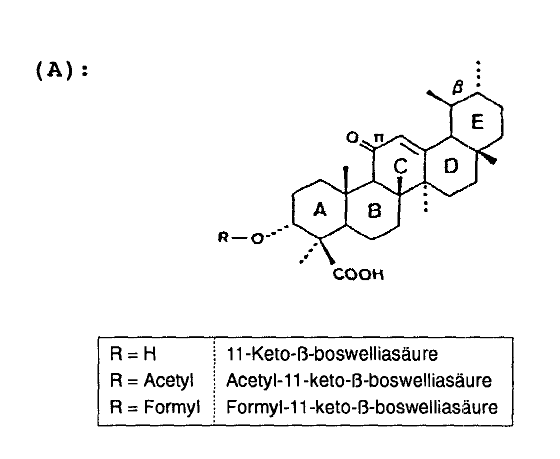

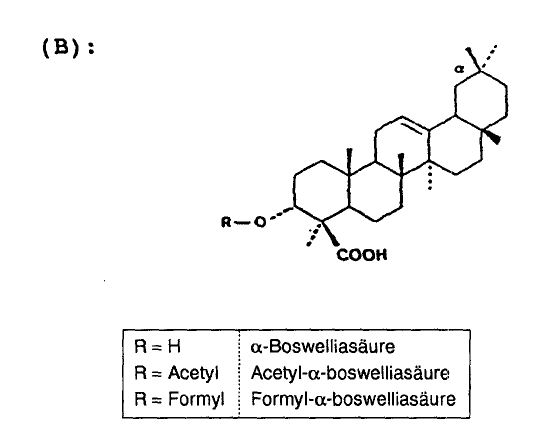

Als Boswelliasäure wird vorzugsweise β-Boswelliasäure verwendet, die nach Literaturangaben aus Boswellia serrata oder anderen bekannten Boswelliasäure enthaltenden Pflanzen isoliert wird. Die β-Boswelliasäure kann geringe Mengen an α-oder γ-Boswelliasäure enthalten. Als physiologisch annehmbare Salze der Boswelliasäure können die Natrium-, Kalium-, Ammonium-, Calciumsalze verwendet werden. Als Derivate der Boswelliasäure können niedere Alkylester, die durch Veresterung der Carboxylgruppe mit einem C₁-C₆-Alkohol erhalten werden, vorzugsweise der Methylester, oder Ester, die durch Veresterung der Hydroxylgruppe mit einer physiologisch verträglichen Carbonsäure erhalten werden, verwendet werden. Bevorzugte Derivate sind β-Boswelliasäureacetat, β-Boswelliasäureformiat, β-Boswelliasäuremethylester, Acetyl-β-boswelliasäure, Acetyl-11-keto-β-boswelliasäure und 11-Keto-β-boswelliasäure.

Erfindungsgemäß ist es weiterhin möglich, eine Boswelliasäure enthaltende pflanzliche Zubereitung zu verwenden. Erfindungsgemäß werden Präparate, die aus dem Harz gewonnen werden, verwendet. Besonders bevorzugt sind Weihrauch (Olibanum) und Weihrauchextrakt.

Eine besonders bevorzugte Boswelliasäure enthaltende pflanzliche Zubereitung ist das von der Firma Ayurmedica, Pöcking, vertriebene Phytopharmakon H 15, ein liphophiler Extrakt aus Boswellia serrata. Dieses verschreibungspflichtige Arzneimittel enthält als Wirkstoff einen Trockenextrakt aus Olibanum. Die Handelsprodukte Tablette bzw. Granulat setzen sich wie folgt zusammen:
1 Tablette enthält 400 mg Trockenextrakt aus Olibanum
(4,2 - 5,9:1), Auszugsmittel: Chloroform/Methanol
1 g Granulat enthält 500 mg Trockenextrakt aus Olibanum
(4,2 - 5,9:1), Auszugsmittel: Chloroform/Methanol.

Erfindungsgemäß können natürliche, synthetische Verbindungen und deren Gemische verwendet werden.

Erfindungsgemäß ist es weiterhin möglich, daß die Verwendung zusammen mit anderen chemischen Arzneistoffen und/oder anderen pflanzlichen Arzneimitteln erfolgt.

Erfindungsgemäß wird die Boswelliasäure je nach Bedarf verabreicht. Da sie wenig toxisch ist, ist die Dosierung nicht kritisch und kann in Abhängigkeit von der Schwere der Krankheit, dem Gewicht des zu behandelnden Patienten und der Dauer der Behandlung vom Arzt leicht variiert werden.

Einheitsdosen können beispielsweise ein- bis viermal täglich verabreicht werden. Die exakte Dosis hängt vom Verabreichungsweg, dem zu behandelnden Zustand, dem Gewicht des Patienten etc. ab. Naturgemäß kann es erforderlich sein, Routinevariationen der Dosis, je nach dem Alter und dem Gewicht des Patienten sowie der Schwere des zu behandelnden Krankheitszustandes, vorzunehmen.

Die erfindungsgemäß verwendeten Zubereitungen können in an sich bekannter Weise unter Verwendung eines oder mehrerer pharmazeutisch annehmbarer Träger oder Verdünnungsmittel formuliert werden. Die Zubereitungen können für die intraperitoneale, orale, bukkale, parenterale, rektale, intra-muskuläre, topische, subkutane, intraartikuläre, intravenöse oder intranasale Verabreichung oder in einer für die Verabreichung durch Inhalation oder Insufflation geeigneten Weise formuliert werden. Zubereitungen der Verbindungen für die orale Verabreichung sind bevorzugt.

Die pharmazeutischen Zubereitungen können in Form von Tabletten, Dragees, Kapseln, Lösungen, Emulsionen, Salben Cremes, Inhalationspräparaten, Aerosolen oder Suppositorien erfolgen.

Die pharmazeutischen Zubereitungen für die orale Verabreichung können in Form von beispielsweise Tabletten oder Kapseln, die nach an sich bekannten Verfahren mit pharmazeutisch annehmbaren Verdünnungsmitteln, wie Bindemitteln (zum Beispiel vorgelatinierter Maisstärke, Polyvinylpyrrolidon oder Hydroxypropylmethylcellulose), Füllstoffen (zum Beispiel Lactose, Saccharose, Mannit, Maisstärke, mikrokristalline Cellulose oder Calciumhydrogenphosphat); Schmiermitteln (zum Beispiel Stearinsäure, Polyethylenglykol, Magnesiumstearat, Talk oder Siliciumdioxid); Desintegrationsmitteln (zum Beispiel Kartoffelstärke, Natriumstärkeglykolat oder Natriumcarboxymethylcellulose); oder Benetzungsmitteln (zum Beispiel Natriumlaurylsulfat), hergestellt werden, vorliegen. Die Tabletten können nach an sich bekannten Verfahren überzogen werden. Flüssige Zubereitungen für die orale Verabreichung können in Form von beispielsweise wäßrigen oder öligen Lösungen, Sirupen, Elixieren, Emulsionen oder Suspensionen vorliegen, oder sie können als Trockenprodukt für die Konstitution mit Wasser oder einem anderen geeigneten Träger vor der Verwendung vorliegen. Solche flüssigen Zubereitungen können nach an sich bekannten Verfahren mit pharmazeutisch annehmbaren Zusatzstoffen hergestellt werden, wie Suspensionsmitteln (zum Beispiel Sorbitsirup, Cellulosederivaten, Glucose/Zucker-Sirup, Gelatine, Aluminiumstearatgel oder hydrierten genießbaren Fetten); Emulgiermitteln (zum Beispiel Lecithin, Gummi arabicum oder Sorbitan-monooleat); nichtwäßrigen Trägern (zum Beispiel Mandelöl, öligen Estern, Ethylalkohol oder fraktionierten Pflanzenölen); und Konservierungsmitteln (zum Beispiel Methyl- oder Propylp-hydroxy-benzoaten oder Sorbinsäure). Die flüssigen Zubereitungen können auch an sich bekannte Puffer, Geschmacks- bzw. Aromamittel, Farbstoffe und Süßstoffe, je nach Bedarf, enthalten.

Für die parenterale Verabreichung können die Verbindungen für Injektion, bevorzugt intravenöse, intra-muskuläre oder subkutane Injektion, formuliert werden. Zubereitungen für die Injektion können in Eindosenform, zum Beispiel in Ampullen, oder in Mehrfachdosis-Behältern mit einem zugegebenen Konservierungsstoff vorliegen. Die Zubereitungen können in Form von Suspensionen, Lösungen oder Emulsionen in öligen oder wäßrigen Trägern vorliegen und Zubereitungshilfsmittel enthalten, wie Suspendier-, Stabilisier- und/oder Dispersionsmittel, und/oder Mittel zur Einstellung der Tonizität der Lösung enthalten. Alternativ kann der aktive Bestandteil in Pulverform für die Konstitution mit einem geeigneten Träger, zum Beispiel sterilem pyrogenfreien Wasser, vor der Verwendung vorliegen.

Die Verbindungen können ebenfalls als rektale Zubereitungen, wie Suppositorien, formuliert werden, zum Beispiel solche, die an sich bekannte Suppositorien-Grundstoffe, wie Kakaobutter oder andere Glyceride, enthalten.

Für die intranasale Verabreichung können die Verbindungen als flüssige Sprays, in Form von Tropfen oder als Schnupfpulver verwendet werden.

Für die Verabreichung durch Inhalation werden die Verbindungen zweckdienlich in Form eines Aerosolsprays aus einer unter Druck stehenden Packung unter Verwendung geeigneter Treibmittel oder in einer Zerstäubungsvorrichtung abgegeben. Im Falle eines unter Druck stehenden Aerosols wird die Dosiseinheit bestimmt, indem ein Ventil vorgesehen wird, welches eine abgemessene Menge freigibt. Kapseln und Patronen aus beispielsweise Gelatine für die Verwendung in einer Inhalationsvorrichtung oder einer Insufflationsvorrichtung können so zubereitet werden, daß sie ein Pulvergemisch aus einer erfindungsgemäß verwendeten Verbindung und einem geeigneten Pulver-Grundstoff, wie Lactose oder Stärke, enthalten.

Die folgenden Beispiele erläutern die erfindungsgemäße Verwendung.

### Beispiel 1

### Tabletten für die orale Verabreichung

### A. Direkte Kompression

(1)

| | |
|---|---|
| Wirkstoff: Boswelliasäure | 15 - 30 mg/Tablette |
| (bzw. pulverisierte Droge | 0,5 - 1,0 g/Tablette) |
| Magnesiumstearat BP | 0,65 mg/Tablette |
| wasserfreie Lactose | 80 mg/Tablette |

Der Wirkstoff wird mit der wasserfreien Lactose und dem Magnesiumstearat vermischt, und das Gemisch wird gesiebt. Das entstehende Gemisch wird zu Tabletten unter Verwendung einer Tablettiermaschine verpreßt.
(2)

| | |
|---|---|
| Wirkstoff: Boswelliasäure | 15 - 30 mg/Tablette |
| (bzw. pulverisierte Droge | 0,5 - 1,0 g/Tablette) |
| Magnesiumstearat BP | 0,7 mg/Tablette |
| mikrokristalline Cellulose NF | 100 mg/Tablette |

Der Wirkstoff wird gesiebt und mit der mikrokristallinen Cellulose und dem Magnesiumstearat vermischt. Das entstehende Gemisch wird unter Verwendung einer Tablettiermaschine zu Tabletten verpreßt.

### B. Nasse Granulierung

| | |
|---|---|
| Wirkstoff: Boswelliasäure | 15 - 30 mg/Tablette |
| (bzw. pulverisierte Droge | 0,5 - 1,0 g/Tablette) |
| Lactose BP | 150,0 mg/Tablette |
| Stärke BP | 30,0 mg/Tablette |
| vorgelatinierte Maisstärke BP | 15,0 mg/Tablette |
| Magnesiumstearat BP | 1,5 mg/Tablette |

Der Wirkstoff wird durch ein geeignetes Sieb gesiebt und mit der Lactose, der Stärke und der vorgelatinierten Maisstärke vermischt. Geeignete Volumina an gereinigtem Wasser werden zugegeben, und das Pulver wird granuliert. Nach dem Trocknen wird das Granulat gesiebt und mit dem Magnesiumstearat vermischt. Das Granulat wird dann unter Verwendung von Lochstanzen mit geeignetem Durchmesser zu Tabletten verpreßt.

Tabletten anderer Zusammensetzung können hergestellt werden, indem man das Verhältnis von Wirkstoff zu Lactose oder das Kompressionsgewicht ändert und entsprechende Lochstanzen verwendet.

### Beispiel 2

| Kapseln | |
|---|---|
| Wirkstoff: Boswelliasäure | 15 - 30 mg/Kapsel |
| (bzw. granulierte Droge | 0,5 - 1,0 g/Kapsel) |
| freifließende Stärke | 150,00 mg/Kapsel |
| Magnesiumstearat BP | 1,00 mg/Kapsel |

| (fortgesetzt) | |
|---|---|
| Kapseln | |
| Magnesiumstearat BP | 1,00 mg/Kapsel |

Der Wirkstoff wird gesiebt und mit den anderen Bestandteilen vermischt. Das Gemisch wird unter Verwendung einer geeigneten Vorrichtung in Hartgelatinekapseln Nr. 2 gefüllt. Andere Kapseln können hergestellt werden, indem man das Füllgewicht ändert und erforderlichenfalls die Kapselgröße entsprechend ändert.

### Beispiel 3

| Sirup | |
|---|---|
| Saccharose-freie Zubereitung | mg/5 ml Dosis |
| Wirkstoff: Boswelliasäure | 15 - 30 |
| Hydroxypropylmethylcellulose USP (Viskositäts-Typ 4000) | 22,5 |
| Puffer ) | |
| Geschmacksstoff ) | |
| Farbstoff ) | nach Bedarf |
| Konservierungsmittel ) | |
| Süßstoff ) | |
| gereinigtes Wasser auf | 5,0 ml |

Die Hydroxypropylmethylcellulose wird in heißem Wasser dispergiert, abgekühlt und dann mit einer wäßrigen Suspension vermischt, die den Wirkstoff und die anderen Komponenten der Zubereitung enthält. Die entstehende Lösung wird auf ihr Volumen eingestellt und vermischt.

### Beispiel 4

| Suspension | mg/5 ml Dosis |
|---|---|
| Wirkstoff: Boswelliasäure | 15 - 30 |
| (bzw. pulverisierte Droge | 0,5 - 1,0 g) |
| (getrockneter Drogenextrakt entsprechend) | |
| Aluminiummonostearat | 75,00 |
| Süßstoff ) | |
| Geschmacksstoff ) | nach Bedarf |
| Farbstoff ) | |
| fraktioniertes Kokosnußöl auf | 5,00 |

Das Aluminiummonostearat wird in etwa 90% des fraktionierten Kokosnußöls dispergiert. Die resultierende Suspension wird unter Rühren auf 115°C erhitzt und dann abgekühlt. Die Süß-, Geschmacks- und Farbstoffe werden zugesetzt, und der Wirkstoff wird dispergiert. Die Suspension wird mit dem restlichen fraktionierten Kokosnußöl auf das Volumen eingestellt und vermischt.

### Beispiel 5

| Sublinguale Tablette | |
|---|---|
| Wirkstoff: Boswelliasäure | 15 - 30 mg/Tablette |
| (bzw. Drogenextrakt | 0,5 - 1,0 g/Tablette) |
| verpreßbarer Zucker NF | 50,5 mg/Tablette |
| Magnesiumstearat BP | 0,5 mg/Tablette |

Der Wirkstoff wird durch ein geeignetes Sieb gesiebt, mit den anderen Bestandteilen vermischt und unter Verwendung geeigneter Lochstanzen verpreßt. Tabletten anderer Stärke können hergestellt werden, indem man das Verhältnis von Wirkstoff zu Träger oder das Kompressionsgewicht ändert.

### Beispiel 6

| Suppositorien für die rektale Verabreichung | |
|---|---|
| Wirkstoff: Boswelliasäure | 15 - 30 mg |
| Witepsol® H15⁺ auf | 1,0 g |
| ⁺ geeignete Qualität von Adeps solidus Ph.Eur. | |

Eine Suspension des Wirkstoffs in geschmolzenem Witepsol® wird hergestellt und unter Verwendung einer geeigneten Vorrichtung in 1-g-Suppositorienformen eingefüllt.

### Beispiel 7

| Injektion für intravenöse Verabreichung | |
|---|---|
| Wirkstoff: Boswelliasäure | 15 - 30 mg/ml |
| Natriumchlorid-intravenöse Infusion, BP, 0,9% Gew./Vol. auf | 1 ml |
| Ansatzgröße 2500 ml | |

Der Wirkstoff wird in einem Teil der Natriumchlorid-intravenösen Infusion gelöst, die Lösung mit der Natriumchloridintravenösen Infusion auf das Volumen eingestellt und die Lösung gründlich vermischt. Die Lösung wird in klare, Typ 1, 10-ml-Glasampullen eingefüllt und unter Stickstoff im Kopfraum durch Abschmelzen des Glases abgesiegelt. Die Ampullen werden durch Erhitzen im Autoklaven bei 120°C für nicht kürzer als 20 Minuten sterilisiert.

### Beispiel 8

| Patrone für die Inhalation | |
|---|---|
| Wirkstoff (mikronisiert): Boswelliasäure | 15 - 30 mg/Patrone |
| Lactose BP | 25,00 |

Der Wirkstoff wird in einer Strahlmühle zu einem feinen Teilchengrößenbereich mikronisiert und dann mit der Lactose vermischt. Die Pulvermischung wird in Hartgelatinekapseln Nr. 3 eingefüllt.

### Beispiel 9

| Nasenspray | |
|---|---|
| Wirkstoff: Boswelliasäure | 1,5 - 3,0 %/Vol. |
| Konservierungsmittel ) | nach Bedarf |
| Natriumchlorid BP ) | |
| gereinigtes Wasser BP auf | 100 |

| Abgabegewicht | |
|---|---|
| | 100 mg (Äquivalent zu |
| | 7 mg Wirkstoff) |

Der Wirkstoff, der Konservierungsstoff und das Natriumchlorid werden in einem Teil des Wassers gelöst. Die Lösung wird mit Wasser auf das Volumen eingestellt und die Lösung gründlich vermischt.

## Patentansprüche

1. Verwendung reiner Boswelliasäure oder eines physiologisch annehmbaren Salzes, eines Derivats, eines Salzes des Derivats oder einer Boswelliasäure enthaltenden pflanzlichen Zubereitung zur Herstellung eines Arzneimittels für die Behandlung der Zerstörung von Funktionsgewebe bei Krankheiten, die durch gesteigerte Leukozytenelastase- oder Plasminaktivität hervorgerufen werden bzw. die durch Hemmung von normaler Leukozytenelastase- oder Plasminaktivität behandelbar sind, in der Human- oder Veterinärmedizin.

2. Verwendung nach Anspruch 1, **dadurch gekennzeichnet, daß** ein Arzneimittel für die Behandlung der Zerstörung von Funktionsgewebe bei Lungenemphysem, akutem Atemnotsyndrom, Schocklunge, zystischer Fibrose (Mucoviscidose), chronischer Bronchitis, Glomerulonephritis und rheumatischer Arthritis, die durch gesteigerte Leukozytenelastaseaktivität hervorgerufen werden, und bei Tumoren und Tumorenmetastasen, die durch gesteigerte Plasminaktivität hervorgerufen werden, hergestellt wird.

3. Verwendung nach Anspruch 2, **dadurch gekennzeichnet, daß** die Verwendung zur Herstellung eines Arzneimittels für die intraperitoneale, orale, bukkale, rektale, intramuskuläre, topische, subkutane, intraartikuläre, intravenöse oder inhalative Verabreichung erfolgt.

4. Verwendung nach einem der Ansprüche 2 oder 3, **dadurch gekennzeichnet, daß** die Verwendung zur Herstellung eines Arzneimittels in Form von Tabletten, Dragees, Kapseln, Lösungen, Emulsionen, Salben, Cremes, Inhalationspräparaten, Aerosolen oder Suppositorien erfolgt.

5. Verwendung nach mindestens einem der Ansprüche 1 bis 4, **dadurch gekennzeichnet, daß** die Verwendung zusammen mit anderen chemisch reinen Arzneistoffen, und/oder pflanzlichen Arzneimitteln erfolgt.

## Claims

1. Use of pure Boswellia acid or of a physiologically acceptable salt, a derivative, a salt of the derivative or a Boswellia acid-containing plant formulation for the preparation of a medicament for treatment of the destruction of functional tissue in diseases which are caused by increased leukocyte elastase or plasmin activity or can be treated by inhibition of normal leukocyte elastase or plasmin activity in human or veterinary medicine.

2. Use according to claim 1, **characterized in that** a medication for treatment of the destruction of functional tissue in pulmonary emphysema, acute dyspnoea syndrome, shock lung, cystic fibrosis (mucoviscidosis), chronic bronchitis, glomerulonephritis and rheumatic arthritis, which are caused by increased leukocyte elastase activity, and for tumours and tumour metastases, which are caused by increased plasmin activity, is prepared.

3. Use according to claim 2, **characterized in that** the use takes place for preparation of a medicament for intraperitoneal, oral, buccal, rectal, intramuscular, topical, subcutaneous, intraarticular, intravenous or inhalatory administration.

4. Use according to one of claims 2 or 3, **characterized in that** the use takes place for preparation of a medicament in the form of tablets, coated tablets, capsules, solutions, emulsions, ointments, creams, inhalation preparations, aerosols or suppositories.

5. Use according to at least one of claims 1 to 4, **characterized in that** the use takes place together with other chemically pure medicinal substances and/or plant medicaments.

## Revendications

1. Utilisation d'un acide de Boswell pur ou d'un sel acceptable du point de vue physiologique, d'un dérivé, d'un sel du dérivé ou d'une préparation végétale contenant un acide de Boswell pour préparer un médicament pour le traitement de la détérioration des fonctions tissulaires par des maladies, qui sont provoquées par une activité accrue de l'élastase leucocytaire ou de la plasmine ou qui peuvent être traitées par blocage de l'activité normale de l'élastase leucocytaire ou de la plasmine, dans la médecine humaine ou vétérinaire.

2. Utilisation selon la revendication 1, **caractérisée en ce qu'**on prépare un médicament pour le traitement de la détérioration des fonctions tissulaires par l'emphysème pulmonaire, du syndrome de dyspnée aigu, de chocs pulmonaires, de fibroses cystiques (mucoviscidose), de bronchite chronique, de glunoméronéphrite et d'arthrite rhumatismale, qui sont provoqués pa.r une activité accrue de l'élastase leucocytaire, et dans le cas de tumeurs et de métastases tumorales, qui sont provoquées par une activité accrue de la plasmine.

3. Utilisation selon la revendication 2, **caractérisée en ce que** l'utilisation s'effectue pour la production d'un médicament pour l'administration intrapéritonéale, orale, buccale, rectale, intramusculaire, topique, sous-cutanée, intra-articulaire, intraveineuse ou par inhalation.

4. Utilisation selon l'une des revendications 2 ou 3, **caractérisée en ce que** l'utilisation s'effectue pour la préparation d'un médicament sous la forme de comprimés, de dragées, de capsules, de solutions, d'émulsions, de pommades, de crèmes, de préparations pour inhalation, d'aérosols ou de suppositoires.

5. Utilisation selon au moins l'une des revendications 1 à 4, **caractérisée en ce que** l'utilisation s'effectue conjointement avec d'autres substances médicamenteuses purement chimiques et/ou d'autres médicaments d'origine végétale.
